# EUROPEAN PATENT SPECIFICATION

(11) **EP 2 794 610 B1**
(45) Date of publication and mention of the grant of the patent: **09.03.2016**
(21) Application number: 12826589.9
(22) Date of filing: 21.12.2012
(51) Int. Cl.: C07D 475/08

(54) **PROCESSES AND INTERMEDIATES FOR PREPARING PRALATREXATE**
VERFAHREN UND ZWISCHENPRODUKTE ZUR HERSTELLUNG VON PRALATREXAT
PROCÉDÉS ET INTERMÉDIAIRES DE PRÉPARATION DU PRALATREXATE

(30) Priority: 21.12.2011 US 201161578491 P; 06.06.2012 US 201261656161 P
(43) Date of publication of application: 29.10.2014
(73) Proprietor: Plus Chemicals SA, 6902 Paradiso (CH); Tiseni, Paolo, Simone, 28100 Novara (US); Galluzzo, Christian, 28100 Novara (IT); Canavesi, Augusto, 22070 Locate Varesino (IT); Biljan, Tomislav, 48260 Krizevci (HR)
(72) Inventor: TISENI, Paolo, Simone, 28100 Novara (IT); GALLUZZO, Christian, CAP 28100 Novara (IT); CANAVESI, Augusto, 22070 Locate Varesino (IT); BILJAN, Tomislav, 48260 Krizevci (HR)
(74) Representative: D Young & Co LLP
(86) International application number: PCT/US2012/071306
(87) International publication number: WO 2013/096800

(56) References cited:
- WO-A1-2011/096947
- DEGRAW J I ET AL: "SYNTHESIS AND ANTITUMOR ACTIVITY OF 10-PROPARGYL-10-DEAZAAMINOPTERIN", JOURNAL OF MEDICINAL CHEMISTRY, AMERICAN CHEMICAL SOCIETY, US, vol. 36, 1 January 1993 (1993-01-01), pages 2228-2231, XP000941891, ISSN: 0022-2623, DOI: 10.1021/JM00067A020 cited in the application

## Description

### FIELD OF THE INVENTION

The present invention encompasses processes for preparing and purifying Pralatrexate, intermediates in these processes, and salts and solid state forms of the Pralatrexate intermediates.

### BACKGROUND OF THE INVENTION

Pralatrexate, +-(2*S*)-2-[[4-[(1*RS*)-1-[(2,4-diaminopteridin-6-yl)methyl]but-3-ynyl]benzoyl]amino]pentandioic acid, also referred to as 10-propargyl-10-deazaaminopterin, is an anti-cancer drug having the following formula: 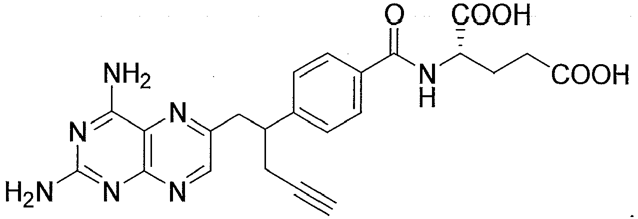 Pralatrexate is approved for a treatment for patients with relapsed or refractory peripheral T-cell lymphoma. It is an antifolate and acts as an inhibitor of dihydrofolate reductase.

Pralatrexate is disclosed in several documents such as DeGraw et al., (J. Med. Chem, 1993, 36, 2228), US 6,028,071, US 5,354,751, EP 0944389, EP 1891957 and WO 98/02163. US 6,028,071 discloses Pralatrexate and a preparation thereof, as described in the following reaction scheme:

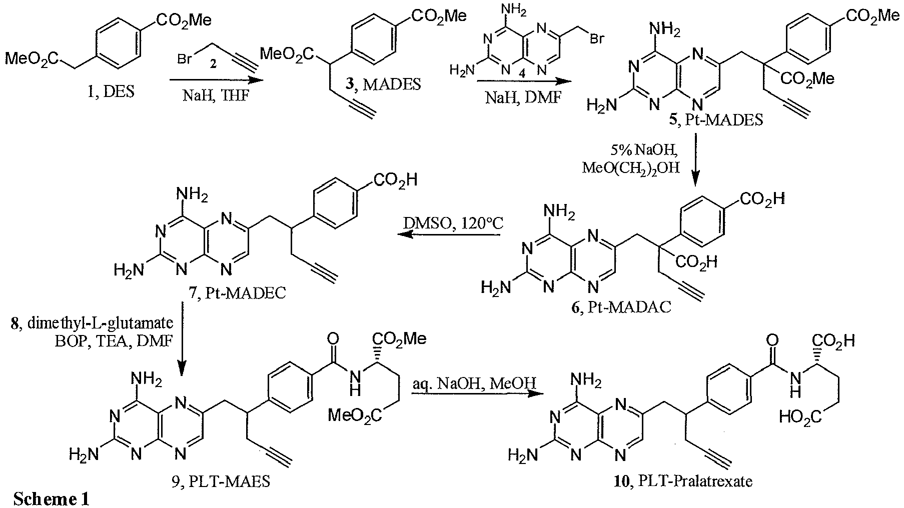

The above described product requires purification of the intermediate and of the product by chromatography. There is a need to provide efficient processes for preparation and purification of Pralatrexate.

### SUMMARY OF THE INVENTION

In one embodiment, the present invention provides isolated Pt-lactone (compound 7b), which is an impurity in the preparation of Pralatrexate, of the following formula:

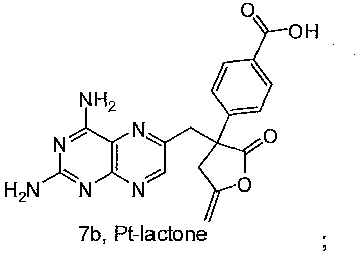

In another embodiment, the present invention provides processes for preparing and purifying the compounds: Pt-MADEC (compound 7), Pt-MADAC (compound 6) and Pt-MADES (compound 5), which are intermediates in the synthesis of Pralatrexate.

In yet another embodiment, the present invention provides salts of Pt-MADEC, of Pt-MADES, of Pt-MADAC and of PLT-ES, and solid state forms of those salts.

In one embodiment, the present invention provides a process for preparing Pralatrexate comprising: preparing any of the above described intermediates of Pralatrexate or crystalline forms thereof, including: Pt-MADEC or salts thereof, Pt-MADAC and Pt-MADES, according to the processes of the present invention, and converting it to Pralatrexate.

In another embodiment, the present invention provides the use of any of the above described intermediates of Pralatrexate or crystalline forms thereof, including: Pt-MADEC or salts thereof, Pt-MADAC and Pt-MADES, for preparing Pralatrexate and Pralatrexate intermediates.

In another embodiment, the invention provides certain salts of Pralatrexate, e.g., sodium, potassium and lithium salts, and crystalline forms of these salts.

### BRIEF DESCRIPTION OF THE FIGURES

Figure 1 shows an X-ray powder diffraction pattern of a crystalline form of Pt-MADEC potassium salt.
Figure 2 shows an X-ray powder diffraction pattern of a crystalline form of Pt-MADEC lithium salt.
Figure 3 shows an X-ray powder diffraction pattern of a crystalline form of Pt-MADEC sodium salt.
Figure 4 shows an X-ray powder diffraction pattern of crystalline form I of Pt-MADES.
Figure 5 shows an X-ray powder diffraction pattern of crystalline form II of Pt-MADES.
Figure 6 shows an X-ray powder diffraction pattern of a crystalline form of Pt-MADAC.
Figure 7 shows an X-ray powder diffraction pattern of a crystalline form of 10-propargyl-10-deazaaminopterin diethyl ester ("PLT-ES" or "compound 9B")
Figure 8 shows an X-ray powder diffraction pattern of a crystalline form of Pralatrexate di-sodium salt (Na-PLT, compound 10a).

### DETAILED DESCRIPTION OF THE INVENTION

The present invention provides Pt-lactone (compound 7b), an impurity formed in the preparation of Pralatrexate, in isolated form. The invention also provides new processes for purifying Pt-MADEC (compound 7), Pt-MADAC (compound 6) and Pt-MADES (compound 5), which are intermediates in the synthesis of Pralatrexate. The present invention also provides solid state forms of Pt-MADEC salts, of Pt-MADES, of Pt-MADAC and of PLT-ES, as well as certain salts of Pralatrexate, e.g., disodium salt and crystalline forms of these salts.

As used herein, the terms "Pt-MADES" and "compound 5" refer to the compound, 10-propargyl-10-carbomethoxy-4-deoxy-4-amino-10-deazapteroic acid methyl ester. Compound 5 can alternatively be named as methyl 4-(2-((2,4-diamino-pteridin-6-yl)methyl)-1-methoxy-1-oxopent-4-yn-2-yl)benzoate.

As used herein, the terms "Pt-MADAC" and "compound 6" refer to the compound, 10-propargyl-10-carboxy-4-deoxy-4-amino-10-deazapteroic acid. Compound 6 can alternatively be named as 4-(2-carboxy-1-(2,4-diaminopteridin-6-yl)pent-4-yn-2-yl)benzoic acid.

As used herein, the terms "hydro-Pt-MADAC" and "compound 6a" refer to the compound, 10-propargyl-10-carboxy-10-deazapteroic acid. Compound 6a can alternatively be named as 4-(1-(2-amino-4-hydroxypteridin-6-yl)-2-carboxypent-4-yn-2-yl)benzoic acid.

As used herein, the terms "Pt-NADAC" and "compound 6c" refer to the compound, 10-carboxy-4-deoxy-4-amino-10-deazapteroic acid.

As used herein, the terms "Pt-MADEC" and "compound 7" refer to the compound, 10-propargyl-4-deoxy-4-amino-10-deazapteroic acid.

As used herein, the terms "hydro-Pt-MADEC" and "compound 7a" refer to the compound, 10-propargyl-10-deazapteroic acid.

As used herein, the terms "Pt-lactone" and "compound 7b" refer to the compound, 4-(3-((2,4-diaminopteridin-6-yl)methyl)-5-methylene-2-oxotetrahydro-furan-3-yl)benzoic acid.

As used herein, the terms "Pt-NADEC" and "compound 7c" refer to the compound, 4-deoxy-4-amino-10-deazapteroic acid.

As used herein, the terms "PLT-MAES" and "compound 9" refer to the compound, 10-propargyl-10-deazaaminopterin dimethyl ester.

As used herein, the terms "PLT-ES" and "compound 9B" refer to the compound 10-propargyl-10-deazaaminopterin ethyl ester.

As used herein, the terms "Na-PLT ", "compound 10a", and Pralatrexate disodium refer to the compound (2*S*)-2-[[4-[(1*RS*)-1-[(2,4-diaminopteridin-6-yl)methyl]but-3-ynyl]benzoyl]amino]pentanedioic acid disodium salt

As used herein, the terms "PLT", Pralatrexate and "compound 10" refer to the compound, (2*S*)-2-[[4-[(1*RS*)-1-[(2,4-diaminopteridin-6-yl)methyl]but-3-ynyl]benzoyl]amino]pentanedioic acid

As used herein, the terms "K-Pt-MADEC" and "compound 11" refer to the compound, potassium 10-propargyl-4-deoxy-4-amino-10-deazapteroate.

As used herein, the terms "hydro-K-Pt-MADEC" and "compound 11 a" refer to the compound, potassium 10-propargyl-l 0-deazapteroate.

As used herein, the terms "K-Pt-lactone-open" and "compound 11b" refer to the compound, potassium 4-(2-carboxylato-1-(2,4-diaminopteridin-6-yl)-4-oxopentan-2-yl)benzoate.

As used herein, the terms "K-Pt-NADEC" and "compound 11c" refer to the compound, potassium 4-deoxy-4-amino-10-deazapteroate.

As used herein, the terms "K-Pt-NADAC" and "compound 11d" refer to the compound, potassium 10-carboxy-4-deoxy-4-amino-10-deazapteroate.

As used herein, the terms "Na-Pt-MADEC" and "compound 12" refer to the compound, sodium 10-propargyl-4-deoxy-4-amino-10-deazapteroate.

As used herein, the terms "hydro-Na-Pt-MADEC" and "compound 12a" refer to the compound, sodium 10-propargyl-10-deazapteroate.

As used herein, the terms "Na-Pt-lactone-open" and "compound 12b" refer to the compound, sodium 4-(2-carboxylato-1-(2,4-diaminopteridin-6-yl)-4-oxopentan-2-yl)benzoate.

As used herein, the terms "Li-Pt-MADEC" and "compound 13" refer to the compound, lithium 10-propargyl-4-deoxy-4-amino-10-deazapteroate.

As used herein, the terms "hydro-Li-Pt-MADEC" and "compound 13a" refer to the compound, lithium 10-propargyl-10-deazapteroate.

As used herein, the terms "Li-Pt-lactone-open" and "compound 13b" refer to the compound, lithium 4-(2-carboxylato-1-(2,4-diaminopteridin-6-yl)-4-oxopentan-2-yl)benzoate.

A thing, *e.g.,* a reaction mixture, may be characterized herein as being at, or allowed to come to "room temperature (often abbreviated "RT"). This means that the temperature of the thing is close to, or the same as, that of the space, *e.g.,* the room or fume hood, in which the thing is located. Typically, room temperature is from about 20°C to about 30°C, or about 22°C to about 27°C, or about 25°C.

A process or step may be referred to herein as being carried out "overnight." This refers to a time interval, e.g., for the process or step, that spans the time during the night, when that process or step may not be actively observed. This time interval is from about 8 to about 20 hours, or about 10-18 hours, typically about 16 hours.

As used herein, the term "reduced pressure" refers to a pressure of about 10 mbar to about 50 mbar.

As used herein, the terms "vol." or "volume" can be used to refer to ml per gram of a solid compound such as Pralatrexate. For example, a statement that 0.5 g of Pralatrexate is dissolved in ten volumes of a Solvent X would be understood to mean that the 0.5 g of Pralatrexate was dissolved in 5 ml of Solvent X.

As used herein, the term "isolated" in reference to Pt-MADEC salts, Pt-MADES, Pt-MADAC, Pt-lactone, PLT-ES, and Pralatrexate sodium, potassium and lithium salts of the present invention, corresponds to Pt-MADEC salts, Pt-MADES, Pt-MADAC, PLT-ES, Pralatrexate sodium, potassium and lithium salts and crystalline forms thereof, or to Pt-lactone that are physically separated from the reaction mixture, wherein they were formed, or, according to some embodiments physically separated into a relatively pure physical state.

As used herein, unless stated otherwise, the XRPD measurements are taken using copper Kα radiation wavelength 1.54184 Å.

A crystal form may be referred to herein as being characterized by graphical data "as shown in," or "as depicted in" a Figure. Such data include, for example, powder X-ray diffractograms and solid state NMR spectra. The skilled person will understand that such graphical representations of data may be subject to small variations, *e.g*., in peak relative intensities and peak positions due to factors such as variations in instrument response and variations in sample concentration and purity, which are well known to the skilled person. Nonetheless, the skilled person would readily be capable of comparing the graphical data in the Figures herein with graphical data generated for an unknown crystal form, and confirm whether the two sets of data are characterizing the same crystal form or two different crystal forms. A crystal form of Pt-MADEC salts, Pt-MADES, Pt-MADAC, PLT-ES, or Pralatrexate sodium, potassium or lithium salts referred to herein as being characterized by graphical data "as shown in," or "as depicted in" a Figure will thus be understood to include any crystal forms of the Pt-MADEC salts, Pt-MADES, Pt-MADAC, PLT-ES or Pralatrexate sodium, potassium or lithium salts characterized with the graphical data having such small variations, as are well known to the skilled person, in comparison with the Figure.

In some embodiments, solid state forms of the compounds disclosed herein, e.g., Pt-MADEC salts, Pt-MADES, Pt-MADAC, PLT-ES and Pralatrexate sodium, potassium or lithium salts described herein are substantially free of any other polymorphic forms of Pt-MADEC salts, or of specified polymorphic forms of Pt-MADEC salts, Pt-MADES, Pt-MADAC, PLT-ES or Pralatrexate sodium, potassium or lithium salts, respectively. In any embodiment of the present invention, by "substantially free" is meant that the forms of the present invention contain 20% (w/w) or less, 10% (w/w) or less, 5% (w/w) or less, 2% (w/w) or less, particularly 1% (w/w) or less, more particularly 0.5% (w/w) or less, and most particularly 0.2% (w/w) or less of any other polymorphs of Pt-MADEC salts, or of a specified polymorph of Pt-MADEC salts, Pt-MADES or Pt-MADAC. In other embodiments, the polymorphs of Pt-MADEC salts, Pt-MADES, Pt-MADAC, PLT-ES or Pralatrexate sodium, potassium or lithium salts of the invention contain from 1% to 20% (w/w), from 5% to 20% (w/w), or from 5% to 10% (w/w)) of any other polymorphs or of a specified polymorph of Pt-MADEC salts, Pt-MADES, Pt-MADAC, PLT-ES or Pralatrexate sodium, potassium or lithium salts, respectively.

The present invention relates to the isolated compound, Pt-lactone (compound 7b), an impurity in the preparation of Pralatrexate, of the following formula:

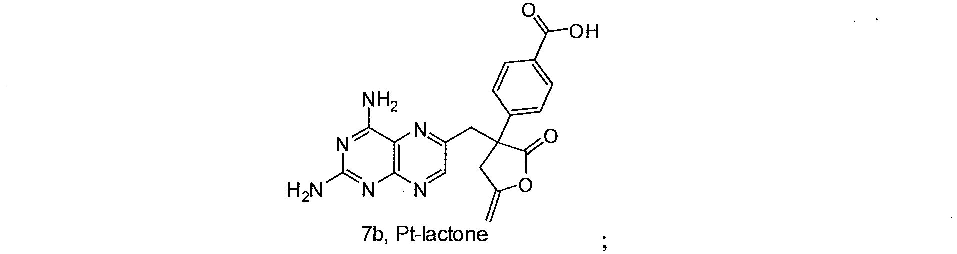

The Pt-lactone impurity may be obtained by decarboxylation of Pt-MADAC (compound 6) to obtain Pt-MADEC (compound 7). The Pt-lactone impurity as well as other impurities such as Pt-NADEC (compound 7c) and Pt-NADAC (compound 6c) can transform to the impurity 10-deazaaminopterin, as disclosed in US 6,028,071, as well as to other impurities that can contaminate the final Pralatrexate.

The known processes for the decarboxylation of Pt-MADAC provide compound 7 (Pt-MADEC) with low purity (about 90%) and flash chromatography is required to purify this compound and/or the compounds produced in subsequent synthesis steps, such as compound 9, in order to provide pure Pralatrexate. The present invention provides a process for purifying compound 7 (Pt-MADEC), especially from Pt-lactone, as well as from the other impurities described above. The process for purifying Pt-MADEC comprises:
a) combining Pt-MADEC with a solution of an inorganic base, wherein the solvent is water, a water miscible solvent, or a mixture of water with a suitable water-miscible solvent, to obtain a mixture; and
b) maintaining the mixture to obtain a precipitate of the corresponding Pt-MADEC salt.

Suitable inorganic bases include alkali or alkaline earth metal hydroxides such as KOH, NaOH or LiOH, for example, KOH. The base is used in an amount of from about 0.05 equivalents to any excess, or from about 0.5 to about 2 equivalents. For example, the base may be used in an amount of about 1.4 equivalents. The mixture can be maintained at a suitable temperature, such as from about -10°C to about 100°C, or from about 0°C to about 40°C, for example, at about room temperature, e.g., about 25°C.

Suitable water miscible solvents for use in this process include, for example, C₁-C₃ alcohols, e.g., methanol, ethanol, and isopropanol; THF; dioxane; acetone and acetonitrile, and mixtures of these water miscible solvents.

The mixture can be maintained for a suitable time period, such as from about 1 minute to about 24 hours, or from about 10 minutes to about 2 hours, for example, for about 60 minutes. The period of maintaining can be followed by cooling the mixture to a temperature such as from about -10°C to about 30°C, for example, about 0°C. The cooling can be maintained for a time period such as from about 1 minute to about 24 hours, or from about 10 minutes to about 2 hours, for example, for about 60 minutes. The precipitate may further be recovered from the mixture, for example, by filtering and drying. The purification procedure may be repeated in order to further increase the purity of Pt-MADEC.

Compound 7 (Pt-MADEC) of the present invention may be prepared by a decarboxylation process. The literature process for this decarboxylation utilizes the solvent dimethyl sulfoxide ("DMSO"). However, this solvent is undesirable because it must be removed under high vacuum conditions. The process of present invention, although still employing high-boiling dipolar aprotic solvents as reaction solvents, does not require the high vacuum conditions that were required in the literature process, thus resulting in an industrially applicable process.

The decarboxylation process of the present invention comprises: combining compound 6 (Pt-MADAC), a dipolar aprotic solvent such as N-methyl-2-pyrrolidone ("NMP"), and optionally a suitable base to obtain a mixture. The dipolar aprotic solvent may be preheated to a suitable temperature, such as from about 70°C to about 200 °C, or from about 100°C to about 150 °C, for example about 120°C. The mixture can be maintained at a suitable reaction temperature such as described above for a time period such as from less than one minute to about 24 hours, or from about 1 minute to about 2 hours, or from about 10 minutes to about 1 hours, for example, for from about 0.5 h to about 1 h. The progress of the reaction can be monitored by HPLC, and the reaction mixture is maintained until all the starting material (Pt-MADAC) is reacted. The maintaining of the reaction temperature may be followed by cooling to a suitable lower temperature such as RT or about 25°C. When the reaction is complete, the reaction mixture may be poured into water, preferably, an excess of water. An excess of water as compared with the amount of dipolar aprotic solvent may range from a volume equal to the volume of dipolar aprotic solvent used, to about 1000-fold excess, for example from about 2-fold to 20-fold excess, or about 10-fold excess.The pH may then be adjusted to a suitable pH, such as from about 3 to about 6, or from about 4 to about 5, for example about 4.5. The pH adjustment may be carried out, for example, by addition of a suitable acid, preferably HCl, to obtain a precipitate. Suitable acids for this step include, for example, carboxylic acids (e.g. acetic acid), and mineral acids (e.g. HCl).

Suitable bases include organic bases such as secondary amines (e.g., diethylamine, dimethylamine), tertiary amines (e.g., triethylamine, tributylamine, diisopropylethyl amine ("DIPEA")) and inorganic bases such as, alkaline metal hydroxides (e.g., NaOH, KOH and LiOH), alkaline earth metal oxides (e.g., MgO, CaO), hydrides (e.g. NaH), carbonates (e.g., K₂CO₃, Na₂CO₃, Cs₂CO₃), bicarbonates (e.g. KHCO₃, NaHCO₃), or alkoxides (e.g., sodium methoxide (NaOMe) or sodium ethoxide (NaOEt)). For example, the base may be a tertiary amine base, such as DIPEA. The base may be in an amount of from about 0.05 equivalents to any excess, or from about 0.1 to about 10 equivalents, for example, in an amount of about 0.75 equivalents. The precipitate may further be recovered from the mixture, for example, by filtering and drying.

When the base used in the purification process was KOH, LiOH or NaOH crystalline forms of Pt-MADEC were obtained. The present invention further provides new crystalline forms of Pt-MADEC salts including: K, Li and Na salts. Those salts and crystalline forms thereof of the present invention may have advantageous properties selected from at least one of: chemical purity, flowability, solubility, dissolution rate, morphology or crystal habit, stability- such as thermal and mechanical stability to polymorphic conversion, stability to dehydration and/or storage stability, low content of residual solvent, a lower degree of hygroscopicity, flowability, and advantageous processing and handling characteristics such as compressibility, and bulk density.

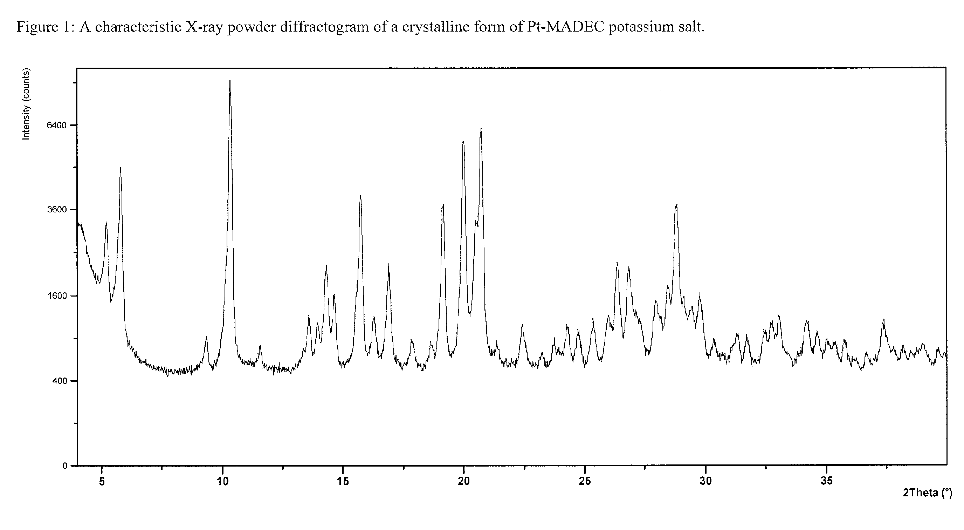

The present invention comprises a crystalline form of Pt-MADEC potassium salt. The crystalline form of Pt-MADEC potassium salt can be characterized by data selected from: an X-ray powder diffraction (XRPD) pattern having peaks at 15.7, 19.1, 20.0, 20.7 and 28.8° 2θ ± 0.2° 2θ; an XRPD pattern substantially as depicted in figure 1; and combinations thereof. The crystalline form of Pt-MADEC potassium salt can be further characterized by an XRPD pattern having any one, two, three, four or five additional peaks selected from: 10.4, 14.3, 16.3, 22.4 and 24.3° 2θ ± 0.2° 2θ.

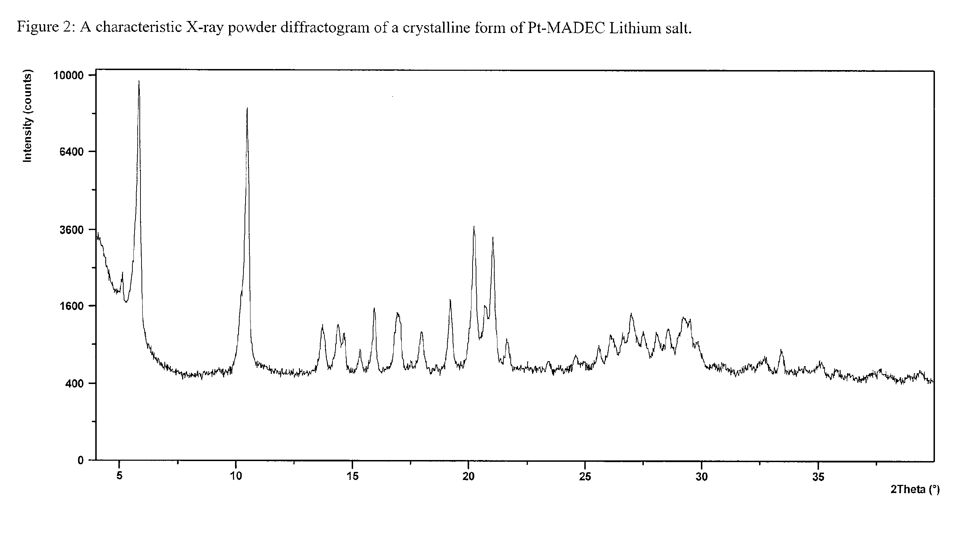

The present invention also comprises a crystalline form of Pt-MADEC lithium salt. The crystalline form of Pt-MADEC lithium salt can be characterized by data selected from: an XRPD pattern having peaks at 13.7, 17.1, 18.0, 21.0 and 26.9° 2θ ± 0.2° 2θ; an XRPD pattern substantially as depicted in figure 2; and combinations thereof. The crystalline form of Pt-MADEC lithium salt can be further characterized by an XRPD pattern having any one, two, three, four or five additional peaks selected from: 10.5, 14.7, 15.3, 19.2 and 25.6° 2θ ± 0.2° 2θ.

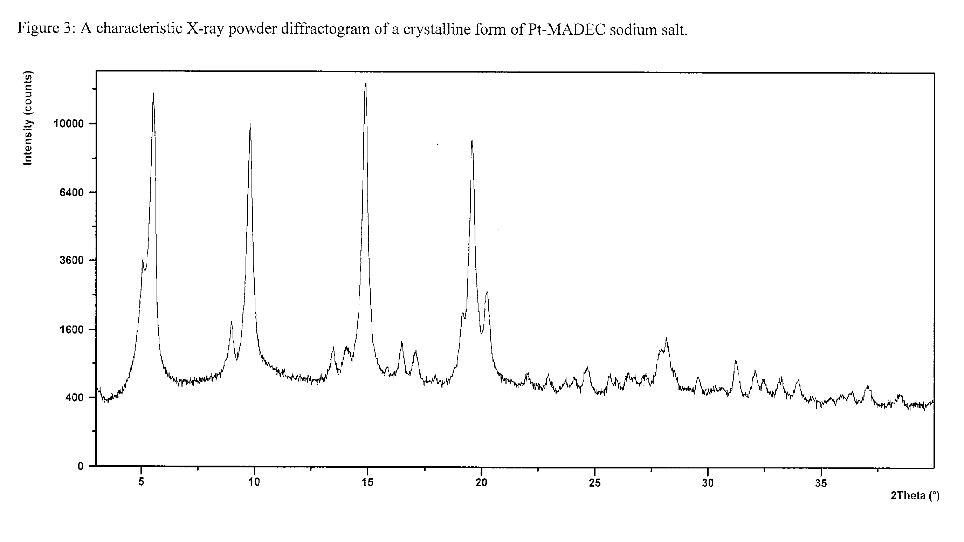

The present invention also comprises a crystalline form of Pt-MADEC sodium salt. The crystalline form of Pt-MADEC sodium salt can be characterized by data selected from: an XRPD pattern having peaks at 5.5, 9.8, 14.9, 19.6 and 20.3° 2θ ± 0.2° 2θ; an XRPD pattern substantially as depicted in figure 3; and combinations thereof. The crystalline Form of Pt-MADEC sodium salt can be further characterized by an XRPD pattern having any one, two, three, four or five additional peaks selected from: 5.1, 9.0, 13.5, 16.5 and 17.1° 2θ ± 0.2° 2θ.

The present invention further provides a process for preparing Pralatrexate comprising preparing Pt-MADEC, or any one of the salts of Pt-MADEC or crystalline forms thereof according to the processes of the present invention, and converting it to Pralatrexate.

The present invention also provides the use of Pt-MADEC or any one of the salts of Pt-MADEC or crystalline forms thereof of the present invention for preparing Pralatrexate.

As mentioned above, Compound 7 may be obtained by decarboxylation of Pt-MADAC (Compound 6). This compound may be obtained in a crystalline form. The present invention also comprises a crystalline form of Pt-MADAC (compound 6).

The crystalline form of compound 6 of the present invention may have advantageous properties selected from at least one of: chemical purity, flowability, solubility, dissolution rate, morphology or crystal habit, stability- such as thermal and mechanical stability to polymorphic conversion, stability to dehydration and/or storage stability, low content of residual solvent, a lower degree of hygroscopicity, flowability, and advantageous processing and handling characteristics such as compressibility, and bulk density.

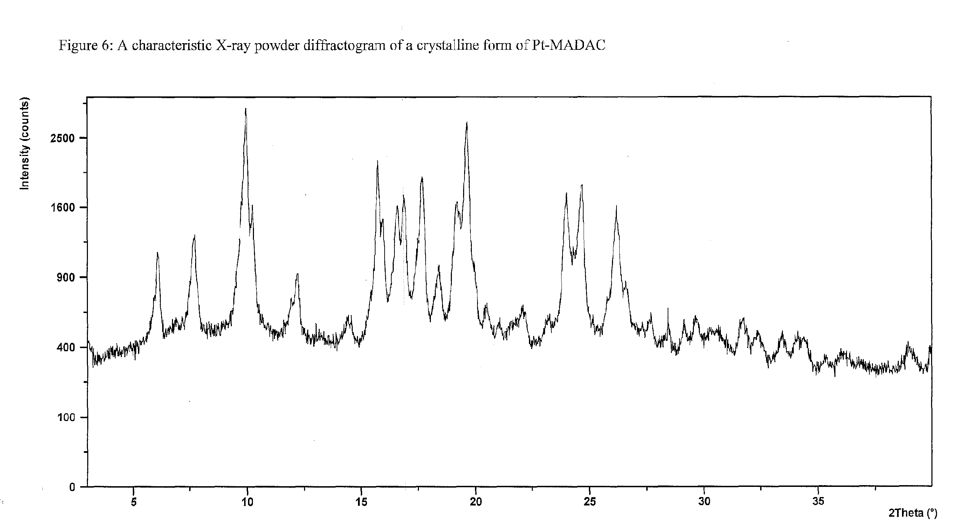

The crystalline form of Pt-MADAC can be characterized by data selected from: an XRPD pattern having peaks at 7.7, 10.0, 15.7, 19.2 and 26.2 ° 2θ ± 0.2° 2θ; an XRPD pattern substantially as depicted in figure 6; and combinations thereof. The crystalline form of Pt-MADAC can be further characterized by an XRPD pattern having any one, two, three, four or five additional peaks selected from: 6.1, 12.2, 17.7, 24.0 and 24.7° 2θ ± 0.2° 2θ.

Compound 6 of the present invention may be obtained by reacting Pt-MADES (compound 5) as exemplified in scheme I above.

The present invention further provides a process for preparing Pralatrexate comprising preparing Pt-MADAC or its crystalline form according to the processes of the present invention and converting it to Pralatrexate.

The present invention also provides the use of the Pt-MADAC or the crystalline form thereof of the present invention for preparing Pralatrexate.

One way to obtain pure Pralatrexate, is to purify the intermediates in its synthesis, such as Compound 5.

The present invention further encompasses a process for purifying Pt-MADES (compound 5) by formation of crystalline forms thereof. The process for purifying Pt-MADES comprises combining Pt-MADES with DMF to obtain a mixture. The DMF may be preheated to a temperature such as from about 25°C to about the reflux temperature, for example to about 120°C. The mixture can be maintained at a temperature such as about 25°C to about the reflux temperature, for example to about 120°C for a time period such as from about 1 minute to about 1 week, or from about 1 minute to about 2 hours, for example about 0.5 h, and then cooled to a temperature such as about RT to obtain a precipitate.

Alternatively, Compound 5 may be purified by a process comprising combining Pt-MADES with a mixture of MeOH and formamide to obtain a mixture. The mixture can be maintained at a temperature such as about 60°C to about 65°C for a time period such as about 2 hours, and then cooled to a temperature such as about RT and maintained at that temperature for about 15 hours, to obtain a precipitate.

The precipitated Pt-MADES may be recovered from the mixture, for example, by filtering; washing; and drying. In some embodiments, the Pt-MADES precipitate may be in a crystalline form.

The present invention comprises crystalline forms of Pt-MADES, designated as Form I and Form II. The crystalline forms of compound 5 of the present invention may have advantageous properties selected from at least one of: chemical purity, flowability, solubility, dissolution rate, morphology or crystal habit, stability-such as thermal and mechanical stability to polymorphic conversion, stability to dehydration and/or storage stability, low content of residual solvent, a lower degree of hygroscopicity, flowability, and advantageous processing and handling characteristics such as compressibility, and bulk density.

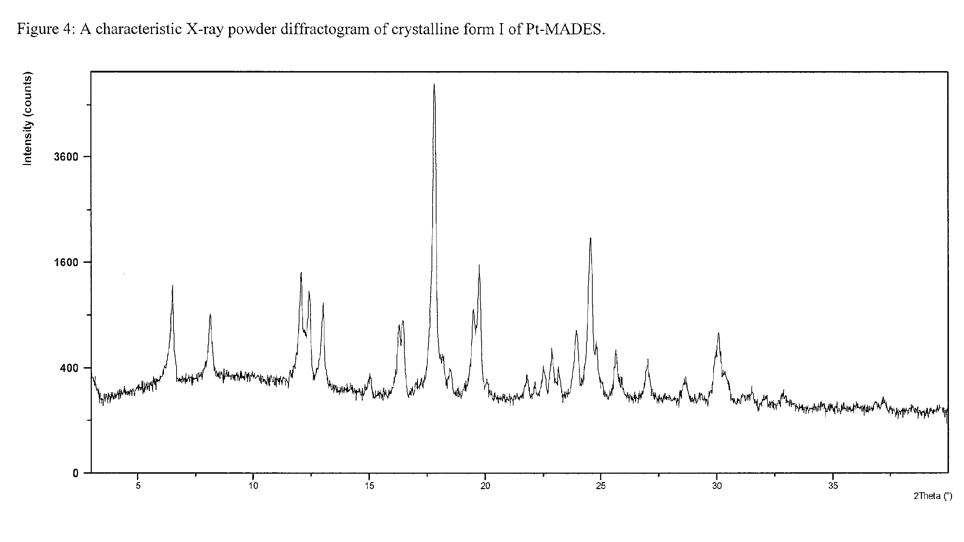

Form I of Pt-MADES can be characterized by data selected from: an XRPD pattern having peaks at 6.5, 12.1, 17.8, 19.8 and 24.6° 2θ ± 0.2° 2θ; an XRPD pattern substantially as depicted in figure 4; and combinations thereof. Form I of Pt-MADES can be further characterized by an XRPD pattern having any one, two, three, four or five additional peaks selected from: 8.1, 12.4, 13.0, 19.5 and 24.0° 2θ ± 0.2° 2θ. Typically, Form I of Pt-MADES is a dimethylformamide ("DMF") solvate.

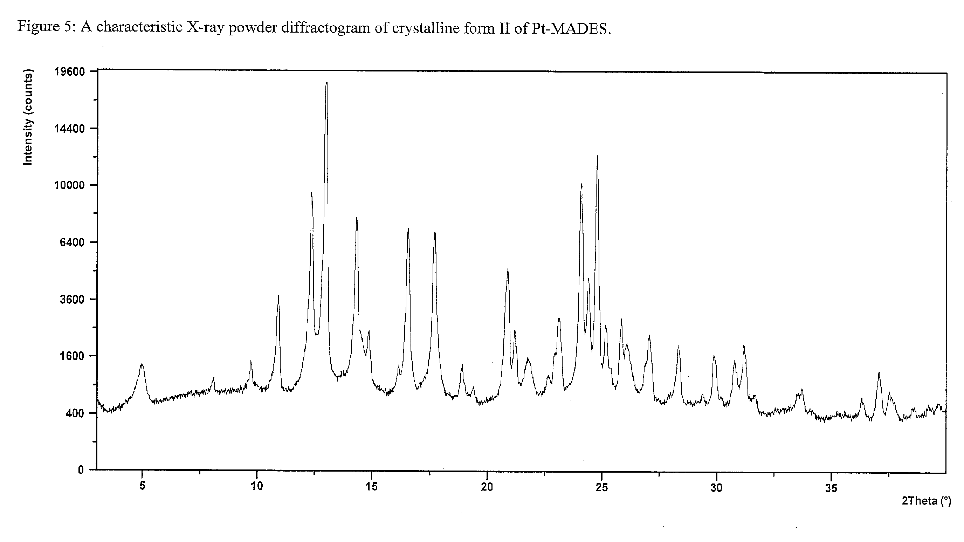

Form II of Pt-MADES can be characterized by data selected from: an XRPD pattern having peaks at 12.4, 13.0, 16.6, 17.7 and 24.8° 2θ ± 0.2° 2θ; an XRPD pattern substantially as depicted in figure 5; and combinations thereof. Form II of Pt-MADES can be further characterized by an XRPD pattern having any one, two, three, four or five additional peaks selected from: 10.9, 20.9, 23.1, 24.1 and 24.4 ° 2θ ± 0.2° 2θ.

The present invention further provides a process for preparing Pralatrexate comprising preparing Pt-MADES or crystalline forms thereof according to the processes of the present invention, and converting it to Pralatrexate.

The present invention also provides the use of the Pt-MADES or crystalline forms thereof of the present invention for preparing Pralatrexate.

Another aspect of the present invention is directed toward a process comprising hydrolyzing PLT-ES (compound 9B) to obtain an alkali salt of Pralatrexate, such as Na-PLT, K-PLT or Li-PLT; in particular Na-PLT (compound 10a).

The starting PLT-ES can be isolated. Preferably the isolated PLT-ES is crystalline.

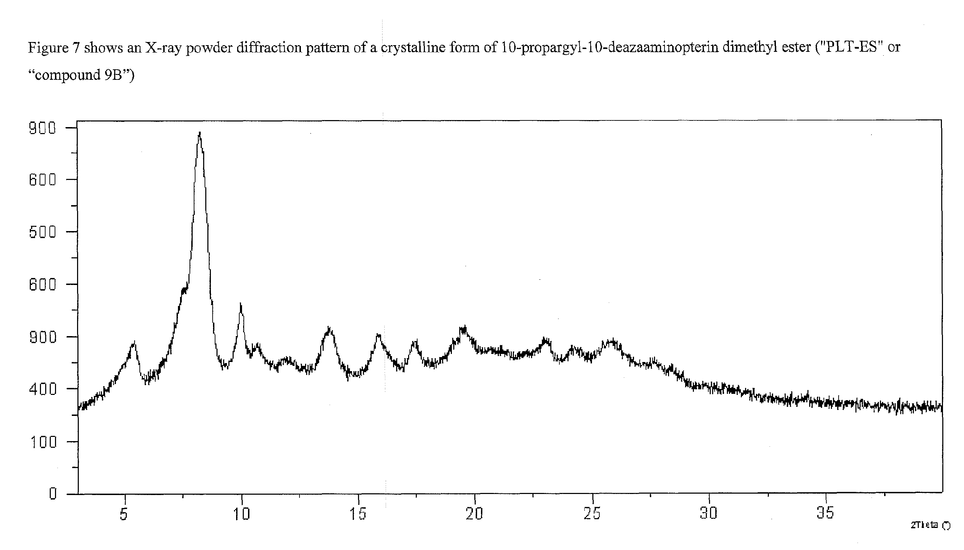

The present invention also provides crystalline PLT-ES (compound 9B). This crystalline form can be characterized by data selected from: an XRPD pattern with peaks at 10.7, 17.5, 19.5 and 23.0 degrees 2-theta ± 0.2 degrees 2-theta; a powder XRD pattern as shown in Figure 7; and combinations thereof.

PLT-ES (compound 9B) can be prepared by a process comprising reacting potassium 10-propargyl-4-deoxy-4-amino-10-deazapteroate ("K-Pt-MADEC" or "compound 11 ") and L-glutamic acid diethyl ester hydrochloride in the presence of 1-hydroxybenzotriazole hydrate ("HOBt") and *N*-(3-Dimethylaminopropyl)-N'-ethylcarbodiimide hydrochloride ("EDC"). This process can be done in the presence of a solvent, such as dichloromethane ("DCM"), 1-methyl-2-pyrrolidinone ("NMP") or a mixture of solvents. The obtained PLT-ES can be isolated in a simple and efficient method, precipitating the compound from a mixture comprising acidic water (i.e. water acidified with an acid, for example hydrochloric acid, prior to the precipitation), and methanol. The precipitation can be achieved by adjusting the pH for a suitable pH in which the product precipitates, i.e., about 4.5.

The hydrolysis of PLT-ES (compound 9B) to obtain an alkali salt of Pralatrexate can comprise slurrying compound 9B in a suitable amount of a suitable solvent and contacting the slurry with a suitable base. Suitable bases for this step include inorganic bases, for example alkali metal or alkaline earth metal hydroxide bases, such as NaOH, KOH or LiOH. The slurrying is typically done by mixing compound 9 and a suitable solvent. Suitable solvents include water miscible solvents, for example C₁-C₃ alcohols, such as methanol, ethanol, or 2-propanol, or mixtures thereof with water. When a mixture of solvent and water is used, a typical ratio for such mixture can be from about 1:1 to about 50:1, or from 2.5:1 to about 20:1, preferably 10:1. Typically, a suitable amount of solvent is an amount in which the compound 9B does not form a clear solution.

As an alternative, the hydrolysis can be carried out in solution and the alkali salt of pralatrexate precipitated subsequently by addition of an anti-solvent. Suitable solvents include water miscible solvents, for example a C₁-C₃ alcohol, such as methanol, ethanol, or 2-propanol.

The above reaction can be done at a suitable temperature, such as from about -100°C (or from above the freezing point of the solvent mixture used) to about 80°C (or up to about the reflux temperature of the solvent mixture used), or from 10°C to 40°C, or about room temperature. The reaction is performed for a suitable period of time, i.e., until the reaction is completed, as can be detected by HPLC

The obtained Pralatrexate salt, in particular compound 10a, can be either isolated from the reaction mixture, or, if desired it can be directly converted to Pralatrexate without isolation.

The present invention provides alkali salts of Pralatrexate such as Na-PLT, K-PLT or Li-PLT; in particular Na-PLT (compound 10a), as well as solid state forms of these salts. Particularly, the present invention provides these salts for use in the preparation of Pralatrexate.

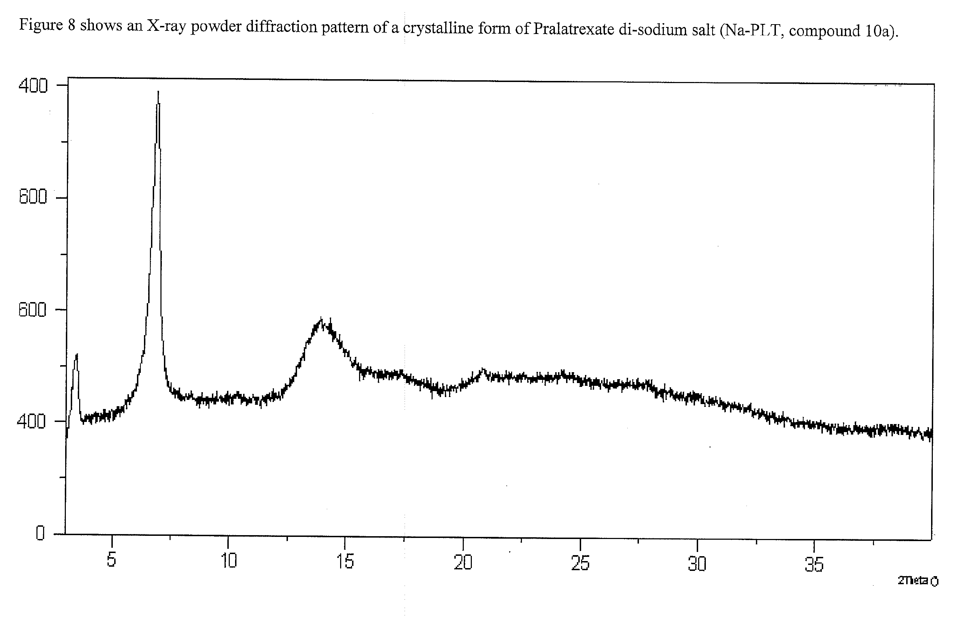

According to one embodiment, the present invention encompasses crystalline Pralatrexate disodium. This crystalline form can be characterized by data selected from: an XRPD pattern with peaks at 3.5, 6.9 and 13.8 degrees 2-theta ± 0.2 degrees 2-theta; an XRPD pattern as shown in Figure 8; and by combinations thereof.

The conversion of PLT salt, in particular compound 10a, to Pralatrexate can typically be done by adjusting the pH to a suitable pH in the presence of an aqueous solvent. A suitable pH can be from about 3 to about 6, or from about 4 to about 5, for example about 4.5. The pH adjustment may be carried out, for example, by addition of an acid such as a carboxylic acid (e.g. acetic acid), a mineral acid (e.g. HCl) and the like to obtain a precipitate. According to one of the embodiments, HCl is used for the pH adjustment. The pH adjustment can be done at a suitable temperature, such as from about -10°C to about 100°C, or from about 10°C to about 60°C, or at about 50°C.

### HPLC method:

| | | | | |
|---|---|---|---|---|
| **Column & Packing** | : | Zorbax ECLIPSE Plus C18 1.8µm, 50x4.6 mm | | |
| **Mobile Phase A** | : | Buffer: Ammonium dihydrogen phosphate 10 mM adjusted to pH 2.0 with H₃PO₄ 85%. | | |
| **Mobile Phase B** | : | Acetonitrile: THF (4:1); | | |

| **Gradient** | : | Time (min) | Mobile Phase A(%) | Mobile Phase B(%) |
|---|---|---|---|---|
| | | 0 | 95 | 5 |
| | | 5 | 95 | 5 |
| | | 15 | 90 | 10 |
| | | 20 | 85 | 15 |
| | | 25 | 80 | 20 |
| | | 40 | 50 | 50 |
| **Flow Rate** | : | 1.0 mL/min; | | |
| **Detector** | : | λ = 340 nm | | |
| **Column Temperature** | : | Room temperature; | | |
| **Injection Volume** | : | 5 µL; | | |
| **Diluent** | : | Methanol with 0.2% HCl 1N; | | |

The following impurities were identified by relative retention time (rrt) versus compound 7 (Pt-MADEC):

| **Compound** | **RRT** |
|---|---|
| 6c, Pt-NADAC | 0.47 |
| 7c, Pt-NADEC | 0.77 |
| 6, Pt-MADAC | 0.86 |
| 7, Pt-MADEC | 1.00 |
| 7a, Hydro Pt-MADEC | 0.96 |
| 7b, Pt-lactone | 1.05 |

### X-ray powder diffraction (XRPD) method:

XRPD was performed on Philips X'Pert PRO powder diffractometer equipped with X'Celerator detector (active length 2⊝ = 2.022°), CuKα radiation, λ = 1.54184 Å at laboratory temperature 22-25°C.

Prior to analysis the samples were gently ground using mortar and pestle in order to obtain a fine powder and applied directly on silicon zero background holder. The scanning parameters were: range: 3-40 degrees two-theta; scan mode: continuous scan; step size: 0.0167°; and time per step: 37 sec.

### EXAMPLES

### Reference examples:

Pt-MADES (compound 5) and Pt-MADAC (compound 6) may be prepared according to procedures disclosed in US 6,028,071, example 1.

### Example 1: Decarboxylation of Pt-MADAC (compound 6)

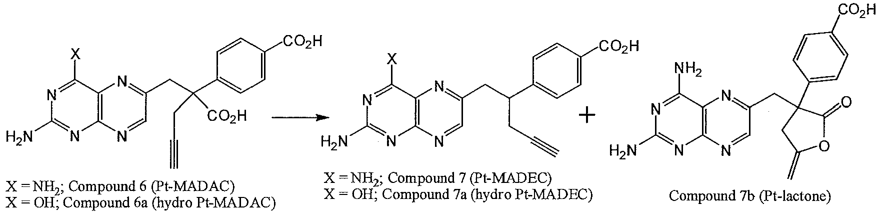

Pt-MADAC (compound 6) (17 g, 43.3 mmol, containing 1.4% of impurity hydro-Pt-MADAC (compound 6a) according to HPLC analysis) was added to N-methyl-2-pyrrolidone (170 mL, 10 Vol.) pre-heated at 120°C. Upon dissolution of the solid, *N,N*-diisopropylethyl amine (5.6 mL, 32.1 mmol) was added. The reaction mixture was stirred at 120 °C for 0.5 h, then cooled down to room temperature and poured into water (1700 mL, 100 Vol.). The pH was adjusted to 4.5 by addition of aq. HCl (16% w/w). A precipitate formed and was isolated by filtration. The collected solid was dried in a drying oven at 45 °C for 18 h to provide Pt-MADEC (compound 7) as a yellow solid (14.6 g, 97% yield, purity 87.1%), containing 7.5% of Pt-lactone (compound 7b) and 1.4% of hydro-Pt-MADEC (compound 7a) according to HPLC analysis.

### Example 2: Decarboxylation of Pt-MADAC (Compound 6) without use of a base

Pt-MADAC (compound 6) (2 g, 5.10 mmol, containing 1.4% of impurity hydro-Pt-MADAC (compound 6a) according to HPLC analysis) was added to N-methyl-2-pyrrolidone (20 mL, 10 Vol.) pre-heated at 120 °C. The reaction mixture was stirred at 120 °C for 1 h, then cooled down to room temperature and poured into water (200 mL, 100 Vol.). The pH was adjusted to 4.5 by addition of aq. HCl (16% w/w) and the precipitate that formed was isolated by filtration. Drying in a drying oven at 45 °C for 18 h furnished Pt-MADEC (compound 7) as a yellow solid (1.65 g, 93% yield, purity 85.8%), containing 6.9% of Pt-lactone (compound 7b) and 1.4% of hydro-Pt-MADEC (compound 7a) according to HPLC analysis.

### Example 3: Purification of Pt-MADEC 7 by precipitation of the corresponding potassium salt

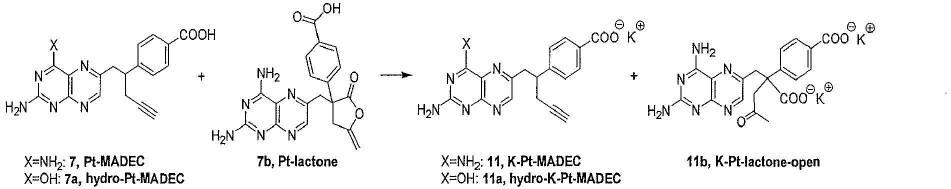

Pt-MADEC (compound 7) (4 g, corresponding to 9.9 mmol of product considering the residual solvent content, prepared according to example 1) was added to aqueous KOH (12.9 mmol of KOH in 40 mL of water, 10 Vol.). The solid dissolved rapidly, and after 0.5 h, the formation of a precipitate started. After 0.5 h at room temperature the reaction mixture was cooled to 0 °C. After 1 h at 0 °C, the precipitate that had formed was isolated by filtration. Drying in a drying oven at 45 °C for 18 h furnished K-Pt-MADEC (compound 11) as a pale yellow solid (2.5 g, 65% yield, purity 99.5%), containing 0.3% of K-Pt-lactone-open (compound 11b) and 0.1% of hydro-K-Pt-MADEC (compound 11a) according to HPLC analysis.

### Example 4: Purification of Pt-MADEC 7 derived from Pt-MADAC containing Pt-NADAC (5%) and Pt-lactone as impurities

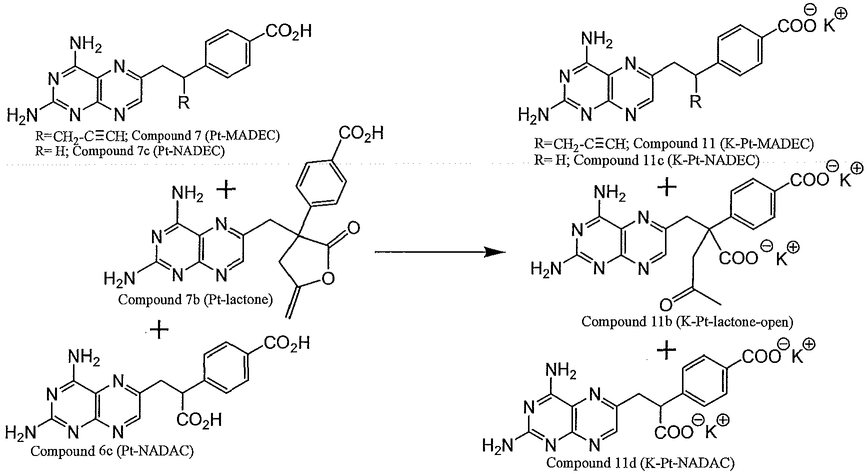

Pt-MADAC (compound 6) (10 g) containing 5% of impurity Pt-NADAC (compound 6c) (according to HPLC analysis) was subjected to the decarboxylation conditions described in Example 1. The isolated Pt-MADEC (compound 7) (8.7 g, 87% yield, purity 83.4%) contained 6.2% of Pt-lactone (compound 7b), 1.6% of Pt-NADEC (compound 7c) and 3.4% of unreacted Pt-NADAC (compound 6c) according to HPLC analysis.

The compound was subjected to the purification conditions described in Example 3, furnishing K-Pt-MADEC (compound 11) (5.3 g, 55% yield, purity 98.5%), containing 0.3% of K-Pt-lactone-open (compound 11b), 0.88% of K-Pt-NADEC (compound 11c) and 0.18% of K-Pt-NADAC (compound 11d) according to HPLC analysis.

### Example 5: Purification of Pt-MADEC 7 by precipitation of the corresponding sodium salt

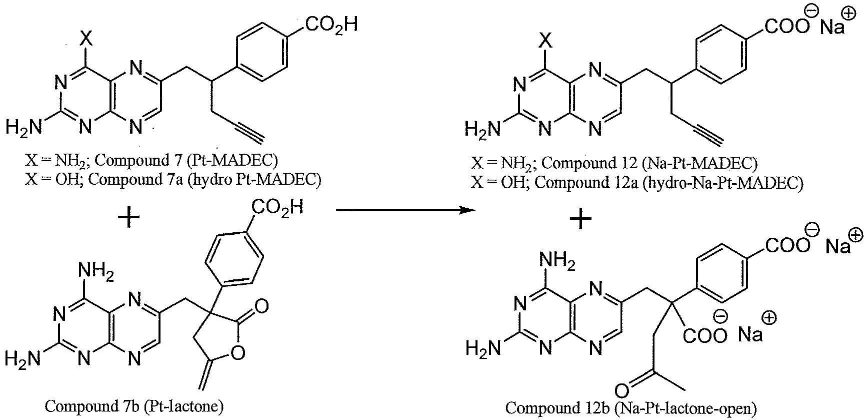

Pt-MADEC (compound 7) (2 g, corresponding to 4.95 mmol of product considering the residual solvent content) was added to aqueous NaOH (6.44 mmol of NaOH in 20 mL of water, 10 Vol.). The solid dissolved rapidly, then after 5 minutes the formation of a precipitate started. After 0.5 h at room temperature, the reaction mixture was cooled to 0 °C. After 1 h at 0 °C the precipitate that had formed was isolated by filtration. The collected solid was then dried in a drying oven at 45 °C for 18 h to provide the Na-Pt-MADEC (compound 12) as a pale yellow solid (1.6 g, 75% yield, purity 96.6%), containing 1.8% of Na-Pt-lactone-open (compound 12b) and 0.3% of hydro-Na-Pt-MADEC (compound 12a) according to HPLC analysis.

### Example 6: Purification of Pt-MADEC 7 by precipitation of the corresponding lithium salt

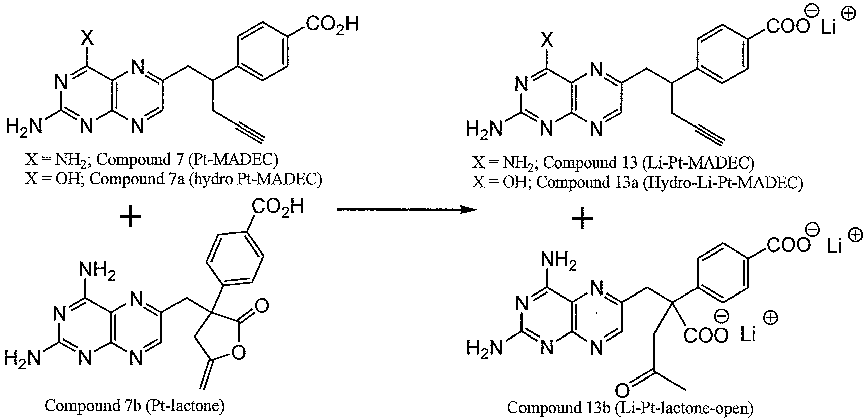

Pt-MADEC (compound 7) (2 g, corresponding to 4.27 mmol of product, considering the residual solvent content), was added to aqueous LiOH (5.55 mmol of LiOH, in 20 mL of water, 10 Vol.). The solid dissolved rapidly, and after 15 minutes the formation of a precipitate started. After 0.5 h at room temperature the reaction mixture was cooled to 0 °C. After 1 h at 0 °C the precipitate that had formed was isolated by filtration. The collected solid was then dried in a drying oven at 45 °C for 18 h to provide Li-Pt-MADEC (compound 13) as a pale yellow solid (1.1 g, 73% yield, purity 97.7%), containing 0.6% of Li-Pt-lactone-open (compound 13b) and 0.9% of hydro-Li-Pt-MADEC (compound 13a) according to HPLC analysis.

### Example 7: Synthesis of Pt-lactone

To a suspension of Pt-MADAC (compound 6) (12.2 g, 31.2 mmol) in acetonitrile (122 mL, 10 Vol.), copper (I) iodide (595 mg, 3.12 mmol) and *N,N-*diisopropylethyl amine (10.9 mL, 62.2 mmol) were added. The reaction mixture was heated to reflux and stirred at reflux for 48 h., and then cooled to room temperature. The resulting precipitate was filtered and washed with acetonitrile (24 mL, 2 Vol.). The collected solid was then dried in a drying oven at 45 °C for 18 h to provide Pt-lactone (compound 7b) as a brown solid (12.3 g, >100% yield, purity 94.1%).

### Example 8: Purification of Pt-MADES by formation of the corresponding DMF solvate

Pt-MADES (compound 5) (40 g, purity 94.7%) was added to DMF (400 mL, 10 Vol.), pre-heated at 120 °C. After dissolution of the product, the reaction mixture was kept at 120°C for 0.5 h, and then cooled to room temperature. The resulting precipitate was filtered and washed with acetone (2 x 200 mL, 2 x 5 Vol.). Drying in a drying oven at 45 °C for 18 h furnished Pt-MADES (compound 5) as a pale yellow solid (36.4 g, 91% yield, purity 97.5%).

### Example 9: purification of Pt-MADES

Pt-MADES (compound 5) (30 g, purity 94.7%) was suspended in a mixture of MeOH (300 mL, 10 Vol.) and formamide (150 mL, 5 Vol.). The suspension was heated at 60-65°C for 2 h, then cooled to room temperature. Upon stirring at room temperature for 15 h, the resulting precipitate was filtered and washed with methanol (2 x 30 mL, 2 x 1 Vol.). Drying in a drying oven at 50 °C for 18 h furnished Pt-MADES (compound 5) as a pale yellow solid (22.5 g, 75% yield, purity 98.0%).

### Example 10: purification of Pt-MADAC

Pt-MADAC (compound 6) (50 g, purity 94.0%) was suspended in a mixture of MeOH (150 mL, 3 Vol.) and formamide (50 mL, 1 Vol.). The suspension was heated at 60-65 °C for 2 h, and then cooled to room temperature. Upon stirring at room temperature for 15 h, a precipitate formed and was filtered and washed with methanol (2 x 50 mL, 2 x 1 Vol.). The collected precipitate was then dried in a drying oven at 50°C for 18 h. The thus-produced Pt-MADES was then suspended in MeOH (500 mL, 10 Vol.). This suspension was heated at reflux for 1 h, and then cooled to 0°C. The resulting precipitate was filtered and washed with methanol (2 x 50 mL, 2 x 1 Vol.). Drying in a drying oven at 50 °C for 18 h furnished Pt-MADAC (compound 6) as a pale yellow solid (41 g, 82% yield, purity 96.3%).

### Example 11: Preparation of Pralatrexate, sodium salt (PLT-Na) (Compound 10a) by hydrolysis of Pralatrexate ethyl ester (PLT-ES) (Compound 9B)

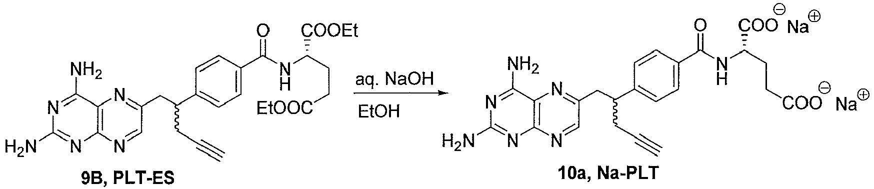

A reactor was charged with EtOH (195 mL) and aqueous NaOH (3.75 M, 19.5 mL, 73 mmol). The mixture was then cooled down to 10 °C. 10-Propargyl-10-deazaaminopterin ethyl ester (PLT-ES, Compound 9B, 13 g, 24.4 mmol) was added, and the temperature was increased to 25 °C over 0.5 h. The resulting suspension was then stirred at 25 °C for 17 h. The solid in the suspension was then isolated by filtration and washed with EtOH (65 mL). The collected solid was then dried in a drying oven at 45 °C for 18 h to provide (2*S*)-2-[[4-[(1*RS*)-1-[(2,4-diaminopteridin-6-yl)methyl]but-3-ynyl]benzoyl]amino]pentanedioic acid disodium salt (Na-PLT; compound 10a) as a pale yellow solid (10.9 g, 86% yield, purity 99.6%).

### Example 12: Preparation of Pralatrexate (Compound 10)

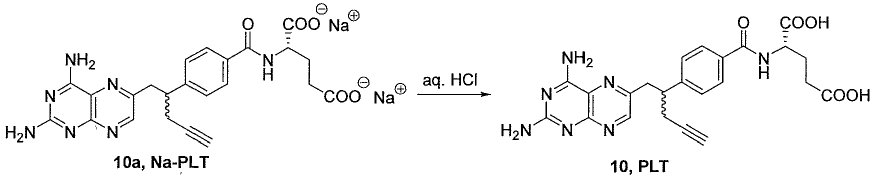

(2*S*)-2-[[4-[(1*RS*)-1-[(2,4-Diaminopteridin-6-yl)methyl]but-3-ynyl]benzoyl]amino]pentanedioic acid disodium salt (Na-PLT, Compound 10a, 10.9 g, 20.9 mmol) was dissolved in water (109 mL). The pH of the solution was adjusted to 4.5 by addition of aqueous HCl 1N. A precipitate formed and was isolated by filtration and washed with water (54 mL). The collected solid was then dried in a drying oven at 45 °C for 17 h to provide Pralatrexate (Compound 10) as a white solid (9.4 g, 81 % yield, and purity 99.7%).

### Example 13: Preparation of Pralatrexate ethyl ester (PLT-ES) (compound 9B)

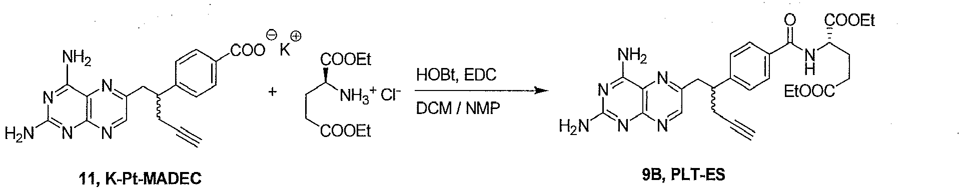

A reactor was charged with potassium 10-propargyl-4-deoxy-4-amino-10-deazapteroate (K-Pt-MADEC, compound 11, 11.1 g, 28.7 mmol). DCM (82 ml) and 1-methyl-2-pyrrolidinone (16.7 ml) were added to obtain a suspension. 1-Hydroxy-benzotriazole hydrate (HOBt, 0.77 g, 5.74 mmol), *N*-(3-dimethylaminopropyl)-N'-ethylcarbodiimide hydrochloride (EDC, 6.60 g, 34.4 mmol) and (*L*)-glutamic acid diethyl ester hydrochloride (7.98 g, 34.4 mmol) were sequentially added. The resulting mixture was stirred at room temperature until HPLC analysis showed reaction completion. DCM was removed under reduced pressure and MeOH (22.2 mL) was added. The reaction mixture was poured into water pre-acidified with aqueous HCl 16% (w/w, 16.9 mL). The pH was adjusted to 4.5 by addition of aqueous NaOH, resulting in the precipitation of the PLT-ES. The solid precipitate was isolated by filtration and dried in oven at 55 °C for 18 h to provide PLT-ES as a pale yellow solid (13 g, 85% yield, and purity 99.0%).

### Example 14: Preparation of crystalline Pralatrexate ethyl ester (compound 9B)

PLT-ES (Compound 9B, 1g) was dissolved in EtOH (15 ml). After a few minutes a precipitate started to form, and the precipitate was isolated by filtration after 45 minutes (0.5g, yellow solid, amorphous form). The mother liquor was left at room temperature overnight, resulting in the precipitation of a yellow solid which was isolated by filtration (0.3g, crystalline form, PXRD is shown on Figure 7).

### Example 15: Hydrolysis of Pralatrexate ethyl ester (compound 9B)

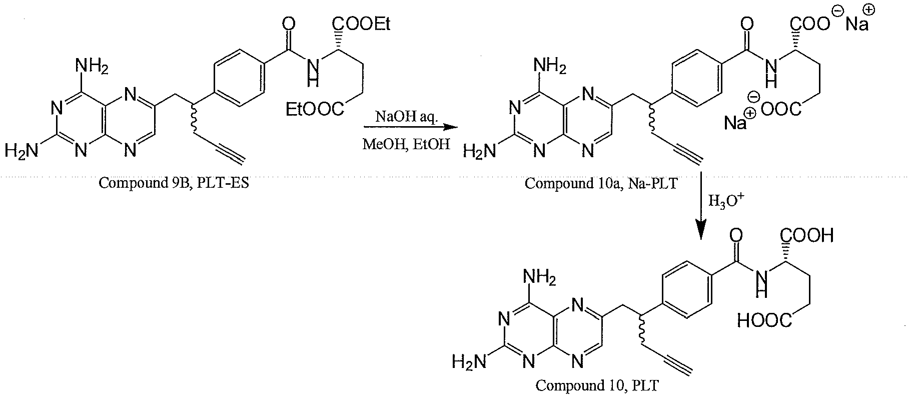

A reactor was charged with MeOH (48 mL), aqueous NaOH (3.75 M, 18.0 mL, 67.5 mmol), and water (6 mL), and the mixture was cooled down to 10 °C. PLT-ES 9B (12 g, 22.5 mmol) was added and the temperature was increased to 25 °C over 1 h. The resulting suspension was stirred at 25 °C for 1 h, and then EtOH (168 mL) was added, resulting in the formation of a precipitate. After stirring for an additional 24 h the solid precipitate was isolated by filtration and washed with EtOH (120 mL). The collected solid was then dried in a drying oven at 60 °C for 18 h to provide Na-PLT 10a (10.5 g, 90% yield, purity 99.8%) as a pale yellow solid.

## Claims

1. The compound 4-(3-((2,4-diaminopteridin-6-yl)methyl)-5-methylene-2-oxotetrahydrofuran-3-yl)benzoic acid (Pt-lactone) in isolated form.

2. A process for purifying the compound, 10-propargyl-4-deoxy-4-amino-10-deazapteroic acid (Pt-MADEC), said process comprising:
a) combining the 10-propargyl-4-deoxy-4-amino-10-deazapteroic acid and a solution of an inorganic base, wherein the solvent is water, a water miscible solvent, or a mixture of water with a water-miscible solvent, to obtain a mixture; and
b) maintaining the mixture to obtain a precipitate of the corresponding Pt-MADEC salt.

3. The process according to claim 2, further comprising recovering the Pt-MADEC salt.

4. The process according to claim 2, wherein the inorganic base is an alkali metal or alkaline earth metal hydroxide, preferably wherein the inorganic base is selected from KOH, NaOH and LiOH.

5. The process according to claim 2, wherein the solvent comprises water.

6. A compound selected from the group consisting of:
- a potassium salt of 10-propargyl-4-deoxy-4-amino-10-deazapteroic acid (K-Pt-MADEC),
- a sodium salt of 10-propargyl-4-deoxy-4-amino-10-deazapteroic acid (Na-Pt-MADEC) and
- a lithium salt of 10-propargyl-4-deoxy-4-amino-10-deazapteroic acid (Li-Pt-MADEC).

7. The potassium salt of 10-propargyl-4-deoxy-4-amino-10-deazapteroic acid (K-Pt-MADEC)according to claim 6 in crystalline form, wherein said crystalline form can be **characterized by** data selected from: an X-ray powder diffraction pattern having peaks at 15.7, 19.1, 20.0, 20.7 and 28.8° 2θ ± 0.2° 2θ; an X-ray powder diffraction pattern substantially as depicted in figure 1; and combinations thereof.

8. The potassium salt of 10-propargyl-4-deoxy-4-amino-10-deazapteroic acid (K-Pt-MADEC)according to claim 7, **characterized by** an X-ray powder diffraction pattern having peaks at 15.7, 19.1, 20.0, 20.7 and 28.8° 2θ ± 0.2° 2θ, and further **characterized by** one, two, three, four or five additional X-ray powder diffraction peaks selected from: 10.4, 14.3, 16.3, 22.4 and 24.3° 2θ ± 0.2° 2θ.

9. The sodium salt of 10-propargyl-4-deoxy-4-amino-10-deazapteroic acid according to claim 6 in crystalline form, wherein said crystalline form can be **characterized by** data selected from: an X-ray powder diffraction pattern having peaks at 5.5, 9.8, 14.9, 19.6 and 20.3° 2θ ± 0.2° 2θ; an X-ray powder diffraction pattern substantially as depicted in figure 3; and combinations thereof.

10. The sodium salt of 10-propargyl-4-deoxy-4-amino-10-deazapteroic acid according to claim 9, **characterized by** an X-ray powder diffraction pattern having peaks at 5.5, 9.8, 14.9, 19.6 and 20.3° 2θ ± 0.2° 2θ, and further **characterized by** one, two, three, four or five additional X-ray powder diffraction peaks selected from: 5.1, 9.0, 13.5, 16.5 and 17.1° 2θ ± 0.2° 2θ.

11. The lithium salt of 10-propargyl-4-deoxy-4-amino-10-deazapteroic acid according to claim 6 in crystalline form, wherein said crystalline form can be **characterized by** data selected from: an X-ray powder diffraction pattern having peaks at 13.7, 17.1, 18.0, 21.0 and 26.9° 2θ± 0.2° 2θ.; an X-ray powder diffraction pattern substantially as depicted in figure 2; and combinations thereof.

12. The lithium salt of 10-propargyl-4-deoxy-4-amino-10-deazapteroic acid according to claim 11, **characterized by** an X-ray powder diffraction pattern having peaks at 5.5, 9.8, 14.9, 19.6 and 20.3° 2θ ± 0.2° 2θ, and further **characterized by** one, two, three, four or five additional X-ray powder diffraction peaks selected from: 10.5, 14.7, 15.3, 19.2 and 25.6° 2θ ± 0.2° 2θ.

13. Pralatrexate disodium salt.

14. Pralatrexate disodium salt according to claim 13 in crystalline form, wherein said crystalline form can be **characterized by** data selected from: an X-ray powder diffraction pattern having peaks at 3.5, 6.9 and 13.8 degrees 2-theta ± 0.2 degrees 2-theta; an X-ray powder diffraction pattern substantially as depicted in figure 8; and combinations thereof.

15. A process for preparing Pralatrexate or a salt thereof, said process comprising purifying the compound, 10-propargyl-4-deoxy-4-amino-10-deazapteroic acid (Pt-MADEC), according to the process of claim 2, and converting said purified 10-propargyl-4-deoxy-4-amino-10-deazapteroic acid to Pralatrexate or a salt thereof.

## Patentansprüche

1. Verbindung 4-(3-((2,4-Diaminopteridin-6-yl)methyl)-5-methylen-2-oxotetrahydrofuran-3-yl)benzoesäure (Pt-Lacton) in isolierter Form.

2. Verfahren zur Aufreinigung der Verbindung 10-Propargyl-4-desoxy-4-amino-10-deazapteroinsäure (Pt-MADEC), wobei das Verfahren Folgendes umfasst:
a) das Kombinieren der 10-Propargyl-4-desoxy-4-amino-10-deazapteroinsäure und einer Lösung einer anorganischen Base, wobei es sich bei dem Lösungsmittel um Wasser, ein mit Wasser mischbares Lösungsmittel oder eine Mischung von Wasser mit einem mit Wasser mischbaren Lösungsmittel handelt, unter Erhalt einer Mischung, und
b) das Aufrechterhalten der Mischung zum Erhalt eines Niederschlags des entsprechenden Pt-MADEC-Salzes.

3. Verfahren nach Anspruch 2, welches weiterhin das Isolieren des Pt-MADEC-Salzes umfasst.

4. Verfahren nach Anspruch 2, wobei es sich bei der anorganischen Base um ein Alkali- oder Erdalkalihydroxid handelt, wobei die anorganische Base vorzugsweise aus KOH, NaOH und LiOH ausgewählt ist.

5. Verfahren nach Anspruch 2, wobei das Lösungsmittel Wasser umfasst.

6. Verbindung, ausgewählt aus der Gruppe bestehend aus:
- einem Kaliumsalz von 10-Propargyl-4-desoxy-4-amino-10-deazapteroinsäure (K-Pt-MADEC),
- einem Natriumsalz von 10-Propargyl-4-desoxy-4-amino-10-deazapteroinsäure (Na-Pt-MADEC) und
- einem Lithiumsalz von 10-Propargyl-4-desoxy-4-amino-10-deazapteroinsäure (Li-Pt-MADEC).

7. Kaliumsalz von 10-Propargyl-4-desoxy-4-amino-10-deazapteroinsäure (K-Pt-MADEC) nach Anspruch 6 in kristalliner Form, wobei die kristalline Form charakterisiert werden kann durch Daten ausgewählt aus: einem Röntgenpulverbeugungsmuster mit Peaks bei 15,7, 19,1, 20,0, 20,7 und 28,8° 2θ ± 0,2° 2θ, einem im Wesentlichen wie in Fig. 1 gezeigten Röntgenpulverbeugungsmuster und Kombinationen davon.

8. Kaliumsalz von 10-Propargyl-4-desoxy-4-amino-deazapteroinsäure (K-Pt-MADEC) nach Anspruch 7, **gekennzeichnet durch** ein Röntgenpulverbeugungsmuster mit Peaks bei 15,7, 19,1, 20,0 20,7 und 28,8° 2θ ± 0,2° 2θ und weiterhin **gekennzeichnet durch** einen, zwei, drei, vier oder fünf zusätzliche Röntgenpulverbeugungspeaks ausgewählt aus: 10,4, 14,3, 16,3, 22,4 und 24,3° 2θ ± 0,2° 2θ.

9. Natriumsalz von 10-Propargyl-4-desoxy-4-amino-10-deazapteroinsäure nach Anspruch 6 in kristalliner Form, wobei die kristalline Form charakterisiert werden kann durch Daten ausgewählt aus: einem Röntgenpulverbeugungsmuster mit Peaks bei 5,5, 9,8, 14,9, 19,6 und 20, 3° 2θ ± 0,2° 2θ, einem im Wesentlichen wie in Fig. 3 gezeigten Röntgenpulverbeugungsmuster und Kombinationen davon.

10. Natriumsalz von 10-Propargyl-4-desoxy-4-amino-deazapteroinsäure nach Anspruch 9, **gekennzeichnet durch** ein Röntgenpulverbeugungsmuster mit Peaks bei 5,5, 9,8, 14,9, 19,6 und 20, 3° 2θ ± 0,2° 2θ und weiterhin **gekennzeichnet durch** einen, zwei, drei, vier oder fünf zusätzliche Röntgenpulverbeugungspeaks ausgewählt aus: 5,1, 9,0, 13,5, 16,5 und 17,1° 2θ ± 0,2° 2θ.

11. Lithiumsalz von 10-Propargyl-4-desoxy-4-amino-10-deazapteroinsäure nach Anspruch 6 in kristalliner Form, wobei die kristalline Form charakterisiert werden kann durch Daten ausgewählt aus: einem Röntgenpulverbeugungsmuster mit Peaks bei 13,7, 17,1, 18,0, 21,0 und 26, 9° 2θ ± 0,2° 2θ, einem im Wesentlichen wie in Fig. 2 gezeigten Röntgenpulverbeugungsmuster und Kombinationen davon.

12. Lithiumsalz von 10-Propargyl-4-desoxy-4-amino-deazapteroinsäure nach Anspruch 11, **gekennzeichnet durch** ein Röntgenpulverbeugungsmuster mit Peaks bei 5,5, 9,8, 14,9, 19,6 und 20, 3° 2θ ± 0,2° 2θ und weiterhin **gekennzeichnet durch** einen, zwei, drei, vier oder fünf zusätzliche Röntgenpulverbeugungspeaks ausgewählt aus: 10,5, 14,7, 15,3, 19,2 und 25,6° 2θ ± 0,2° 2θ.

13. Pralatrexat-Dinatriumsalz.

14. Pralatrexat-Dinatriumsalz nach Anspruch 13 in kristalliner Form, wobei die kristalline Form gekennzeichnet werden kann durch Daten ausgewählt aus: einem Röntgenpulverbeugungsmuster mit Peaks bei 3,5, 6,9 und 13,8 Grad 2-Theta ± 0,2 Grad 2-Theta, einem im Wesentlichen wie in Fig. 8 gezeigten Röntgenpulverdiffraktionsmuster und Kombinationen davon.

15. Verfahren zur Herstellung von Pralatrexat oder einem Salz davon, wobei das Verfahren die Aufreinigung der Verbindung 10-Propargyl-4-desoxy4-amino-10-deazapteroinsäure (Pt-MADEC) gemäß dem Verfahren von Anspruch 2 und die Umwandlung der aufgereinigten 10-Propargyl-4-desoxy-4-amino-10-deazapteroinsäure in Pralatrexat oder ein Salz davon umfasst.

## Revendications

1. Composé d'acide 4-(3-((2,4-diaminoptéridin-6yl)méthyl)-5-méthylène-2-oxotétrahydrofuran-3yl)benzoïque acide (Pt-lactone) sous forme isolée.

2. Procédé de purification du composé, acide 10propargyl-4-désoxy-4-amino-10-déazaptéroïque (PtMADEC), ledit procédé comprenant :
a) la combinaison de l'acide 10-propargyl-4-désoxy-4amino-10-déazaptéroïque et d'une solution d'une base inorganique, où le solvant est l'eau, un solvant miscible dans l'eau, ou un mélange d'eau avec un solvant miscible dans l'eau, pour obtenir un mélange ; et
b) le maintien du mélange pour obtenir un précipité du sel de Pt-MADEC correspondant.

3. Procédé selon la revendication 2, comprenant en outre la récupération du sel de Pt-MADEC.

4. Procédé selon la revendication 2, dans lequel la base inorganique est un hydroxyde de métal alcalin ou de métal alcalino-terreux, de préférence où la base inorganique est choisie parmi KOH, NaOH et LiOH.

5. Procédé selon la revendication 2, dans lequel le solvant comprend l'eau.

6. Composé choisi dans le groupe constitué de :
- un sel de potassium d'acide 10-propargyl-4-désoxy4-amino-10-déazaptéroïque (K-Pt- MADEC),
- un sel de sodium d'acide 10-propargyl-4-désoxy-4amino-10-déazaptéroïque (Na-Pt- MADEC) et
- un sel de lithium d'acide 10-propargyl-4-désoxy-4amino-10-déazaptéroïque (Li-Pt- MADEC).

7. Sel de potassium d'acide 10-propargyl-4-désoxy-4-amino-10-déazaptéroïque (K-Pt-MADEC) selon la revendication 6 sous forme cristalline, ladite forme cristalline pouvant être **caractérisée par** des données choisies parmi : un diagramme de diffraction des rayons X sur poudre ayant des pics à 15,7, 19,1, 20,0, 20,7 et 28,8° 20 ± 0,2° 2θ ; un diagramme de diffraction des rayons X sur poudre sensiblement comme décrit sur la figure 1 ; et des combinaisons de celles-ci.

8. Sel de potassium d'acide 10-propargyl-4-désoxy-4-amino-10-déazaptéroïque (K-Pt- MADEC) selon la revendication 7, **caractérisé par** un diagramme de diffraction des rayons X sur poudre ayant des pics à 15,7, 19,1, 20,0, 20,7 et 28,8° 20 ± 0,2° 2θ, et **caractérisé en outre par** un, deux, trois, quatre ou cinq pics de diffraction des rayons X sur poudre additionnels choisis parmi : 10,4, 14,3, 16,3, 22,4 et 24,3° 2θ ± 0,2° 2θ.

9. Sel de sodium d'acide 10-propargyl-4-désoxy-4-amino-10-déazaptéroïque selon la revendication 6 spis forme cristalline, où ladite forme cristalline peut être **caractérisée par** des données choisies parmi : un diagramme de diffraction des rayons X sur poudre ayant des pics à 5,5, 9,8, 14,9, 19,6 et 20, 3° 2θ ± 0,2° 2θ ; un diagramme de diffraction des rayons X sur poudre sensiblement comme décrit sur la figure 3 ; et des combinaisons de celles-ci.

10. Sel de sodium d'acide 10-propargyl-4-désoxy-4-amino-10-déazaptéroïque selon la revendication 9, **caractérisé par** un diagramme de diffraction des rayons X sur poudre ayant des pics à 5,5, 9,8, 14,9, 19,6 et 20,3° 2θ ± 0,2° 2θ, et **caractérisé en outre par** un, deux, trois, quatre ou cinq pics de diffraction des rayons X sur poudre additionnels choisis parmi : 5,1, 9,0, 13,5, 16,5 et 17,1° 2θ ± 0,2° 2θ.

11. Sel de lithium d'acide 10-propargyl-4-désoxy-4-amino-10-déazaptéroïque selon la revendication 6 sous forme cristalline, où ladite forme cristalline peut être **caractérisée par** des données choisies parmi : un diagramme de diffraction des rayons X sur poudre ayant des pics à 13,7, 17,1, 18,0, 21,0 et 26,9° 2θ ± 0,2° 2θ. ; un diagramme de diffraction des rayons X sur poudre sensiblement comme décrit sur la figure 2 ; et des combinaisons de celles-ci.

12. Sel de lithium d'acide 10-propargyl-4-désoxy-4-amino-10-déazaptéroïque selon la revendication 11, **caractérisé par** un diagramme de diffraction des rayons X sur poudre ayant des pics à 5,5, 9,8, 14,9, 19,6 et 20,3° 2θ ± 0,2° 2θ, et **caractérisé en outre par** un, deux, trois, quatre ou cinq pics de diffraction des rayons X sur poudre additionnels choisis parmi : 10,5, 14,7, 15,3, 19,2 et 25,6° 2θ ± 0,2° 2θ.

13. Sel disodique de pralatrexate.

14. Sel disodique de pralatrexate selon la revendication 13 sous forme cristalline, où ladite forme cristalline peut être **caractérisée par** des données choisis parmi : un diagramme de diffraction des rayons X sur poudre ayant des pics à 3,5, 6,9 et 13,8 degrés 2-thêta ± 0,2 degré 2-thêta ; un diagramme de diffraction des rayons X sur poudre sensiblement comme décrit sur la figure 8 ; et des combinaisons de celles-ci.

15. Procédé de préparation de pralatrexate ou un sel de celui-ci, ledit procédé comprenant la purification du composé, acide 10-propargyl-4-désoxy-4-amino-10-déazaptéroïque (Pt-MADEC), selon le procédé de la revendication 2, et la conversion dudit acide 10-propargyl-4-désoxy-4-amino-10-déazaptéroïque purifié en pralatrexate ou un sel de celui-ci.
